# EUROPEAN PATENT SPECIFICATION

(11) **EP 0 361 983 B1**
(45) Date of publication and mention of the grant of the patent: **15.05.1996**
(21) Application number: 89310066.9
(22) Date of filing: 02.10.1989
(51) Int. Cl.: C12Q 1/68

(54) **RNA template end-linked probe constructs and methods for use**
RNS-Template mit endverbundener Sonde und Verfahren zur Verwendung
Sonde liée à la terminaison d'un moule d'ARN et procédés d'utilisation

(30) Priority: 30.09.1988 US 252243; 22.06.1989 US 370218
(43) Date of publication of application: 04.04.1990
(60) Divisional application: 95114651.3
(73) Proprietor: AMOCO CORPORATION, Chicago Illinois 60680-0703 (US)
(72) Inventor: Stefano, James E., Marlboro, MA 01752 (US)
(74) Representative: Sheard, Andrew Gregory

(56) References cited:
- WO-A-87/06720
- WO-A-90/03446
- BIOTECHNOLOGY, vol. 6, October 1988; P.M. LIZARDI et al., pp. 1197-1202

## Description

This application relates to the detection of organisms in the clinical and food environment and more particularly relates to new RNA template probe constructions used with RNA directed RNA polymerase amplification hybridization assays.

The need to detect low levels of pathogenic organisms in bodily fluids and foodstuffs is of primary importance in the diagnosis, treatment, and prevention of disease. Current approaches to detect such organisms include their isolation and culture in vitro or by direct detection of an analyte specific to the organism. Such analytes may be either the nucleic acid genome of the organism, a ribonucleic acid produced by transcription of its genome during propagation, or proteins or metabolites specifically made by or elicited in the host by the infecting organism.

Methods to detect the presence of specific nucleic acids in a sample are well understood and utilize the same general principle: the high-affinity interaction between one strand of nucleic acid bearing a specific sequence of nucleotides for another strand of nucleic acid possessing the complementary sequence to form a hybrid, or double-stranded structure. The formation of such hybrids can be made to be highly specific ("stringent") by adjustment of the conditions under which this hybridization takes place such that hybridization will not occur unless the sequences are precisely complementary. If total nucleic acid from the sample is immobilized on a solid support such as a nitrocellulose membrane, the presence of a specific "target" sequence in the sample can be determined by the binding of a complementary nucleic acid "probe" which bears a detectable moiety. Such moieties may be radioactive nuclei, fluorescent compounds, or ligands which are recognized by protein conjugates bearing catalytic activities capable of generating colored, luminescent, or fluorescent products. After removal of non-hybridized probe by washing the support, the amount of target is determined by the amount of detectable moiety present.

In practice, the sensitivity of such assays is limited by the number of labelled moieties which one may physically incorporate into the probe nucleic acid. In the case of radioactively-labelled probes, the practical limit is about 10⁴ target molecules. However, to achieve this sensitivity requires probes of very high specific activity which disadvantageously possess a very limited useful lifetime coupled with a detection step (i.e.-autoradiography) requiring several days. Other labelling methods utilizing fluorescent, chemiluminescent, or enzymatic detection, although more rapid, usually do not exceed the sensitivity of isotopically-labelled probes. Since most organisms of clinical interest do not contain more than 50,000 copies of any nucleic acid suitable for use as a target, the utility of such methods is restricted to the detection of large numbers of organisms. The level of infectious agents in clinical specimens or foodstuffs, however, often does not exceed a few (1-10) per sample. Thus, a need exists for methods able to to detect such low levels.

It is one aspect of the present invention to improve the sensitivity of nucleic acid hybridization assays.

One approach for the detection of low levels of DNA utilizes a DNA-dependent DNA polymerase to directly replicate the DNA target to increase its numbers to easily detectable levels. This approach is termed "polymerase chain reaction" (PCR). Saiki, R.K., Scharf, S., Faloona, F., Mullis, K.B., Horn, G.T., Erlich, H.A., and Arnheim, N., "Enzymatic Amplification of Beta-globin Genomic Sequences and Restriction Site Analysis for Diagnosis of Sickle Cell Anemia", Science 230:1350-1354 (1985); Saiki, R.K., Gelfand, D.H., Stoffel, S., Scharf, S.J. Higuchi, R., Horn, G.T., Mullis, K.B., and Erlich, H.A., "Primer-directed Enzymatic Amplification of DNA with a Thermostable DNA Polymerase", Science 239:487-491 (1988); Erlich, H.A., Gelfand, D.H., and Saiki, R.K., "Specific DNA Amplification", Nature 331:461-(1988); and Mullis et al., European Patent Application Nos. 200362 and 201184 (see also U.S. Patents 4,683,195 and 4,683,202). In the PCR method, two DNA oligonucleotides are hybridized to specific, separated sites on the complementary strands of target DNA separated by heat treatment. The annealed probe(s) then are used to prime the synthesis of the complementary strand(s) with a DNA-dependent DNA polymerase to yield two double-stranded copies of the target. After synthesis, the strands in the resulting duplex DNA products are again separated by heat treatment, the oligonucleotide probes (present in vast excess) are reannealed to both the target and the replicated strands, and incubated further with the polymerase. Since both the target DNA and the DNA strands generated by the enzymatic extension of the oligonucleotide probes serve as templates for the primer-directed replication, the amount of specific product DNA doubles with each round until a large number of product strands (for example, one million copies) are generated.

In practice, this technique is limited by the requirement that the target for amplification be DNA (as opposed to RNA), and by the occurrence of false positives generated by hybridization of probes to homologous sites in non-target DNA which fortuitously generate similar replication products. Moreover, although target DNA may be detected with very high sensitivity, the numbers of targets present in the sample is difficult to determine without adding significantly to the complexity of the assay. Since the number of infectious agents is often important in evaluating the treatment protocol for disease, this amplification approach is disadvantageously limited because it provides qualitative rather than quantitative results.

It is another aspect of the present invention to provide methods and probes which may be more advantageously used with quantitative assays.

Another approach to improving the sensitivity of nucleic acid detection is to employ a hybridization probe coupled to or incorporated within an RNA molecule that may be replicated by (i.e.-is a template for) an RNA-directed RNA polymerase. For example, a number of means to generate RNA probes by derivitizing MDV-1 RNA, a 221 nucleotide RNA template for the RNA-dependent RNA polymerase, Qβ replicase, are provided by Chu, B.C.F., Kramer, F.R., and Orgel, L.E., "Synthesis of an Amplifiable Reporter RNA for Bioassays", Nucl. Acids Res. 14: 5591-5603 (1986); Lizardi, P.M., Guerra, C.E., Lomeli, H., Tussie-Luna, I, and Kramer, F.R., "Exponential Amplification of Recombinant-RNA Hybridization Probes", Bio/Technology 6:1197-1203 (October, 1988); and European Patent Application 266,399 (EP Application No. 87903131.8). The replicatable RNA-probe construct may be advantageously employed in a sensitive hybridization assay, such as that described by Ranki, et al.. U.S. 4, 563,419; Soderlund, G.B., U.S. 2,169,403; Stabinsky, U.S. 4,751,177; and Syvanenen, et al.., Nucl. Acids Res. 14:5037-5048. Following hybridization, the RNA-probe chimera associated with the target is replicated to generate amounts of RNA which may be easily detected by variety of means (for example, by fluorescence using a dye such as ethidium bromide or propidium iodide). Since the MDV-1 RNA template for Qβ replicase is doubled in number every 20 seconds in vitro, an exponential increase (for example, a billion-fold) in the number of RNA molecules occurs within a few minutes at a single temperature, and proceeds at that rate until the number of templates exceeds the number of active enzyme molecules in the reaction. After that point, the amount of template RNA increases linearly with time. As a consequence, in reactions given a sufficient period of time to reach this linear phase (for example 15 minutes for 100 molecules), the amount of amplified product RNA will be directly related to the logarithm of the number of template RNAs initially added (Lizardi et al., supra). Since the initial number of template-probes is proportional to the amount of targets, the number of targets present in the sample being examined may be advantageously quantitated over a very wide range in contrast to the so-called PCR amplification techniques.

It is yet another aspect of the present invention to take advantage of the properties of such RNA templates for the RNA dependent, RNA polymerase, Q-beta replicase.

Heretofore, there existed a limited number of means for joining the replicatable RNA to a probe. To be useful, such probe-RNA constructs must either serve directly as templates for the replicase or allow for the removal of the probe moiety prior to replication. In one approach, the probe is coupled to the RNA via a cystamine moiety containing a disulfide (-S-S-) linkage which can be cleaved prior to replication (Chu et al., supra). However, this method suffers from the need for several synthesis steps that increase the cost in labor of producing such probes. In addition and as will be readily recognized by those skilled in the art, such disulfide linkages are subject to premature cleavage by reducing agents (for example, glutathione) which occur naturally in many biological samples.

It is still another aspect of the present invention to avoid the limitations imposed by such disulfide linkage constructions.

In another approach, the probe sequence may be incorporated within the sequence of the replicatable RNA (Lizardi et al., supra). However, the foreign RNA sequence in many such constructs either disadvantageously affects the ability of the RNA to be efficiently replicated, or is spontaneously deleted during replication. For example, among a collection of eight such constructs, two were replicated faithfully, but six either replicated poorly or suffered deletion of the probe sequence during replication. Since such deletion events affect the rate of replication and occur randomly with time, the level of the RNA products obtained in the linear phase of the amplification cannot be used to assess target level. Moreover, for many target organisms, only a limited number of sequences (for example, one or two) may be used as hybridization targets because of a high degree of sequence homology between the target and nucleic acids in uninfected material. Thus, the additional requirement that the probe sequence be stably and efficiently replicated limits the general application of this approach.

It is still yet another aspect of the present invention to provide probe-template RNA constructions and methods which overcome this limitation.

Linking the probe sequence to either the 3′ or 5′ termini of template RNAs via the phosphodiester linkage normally found in RNAs, although simple to accomplish by a variety of means, has been reported to strongly inhibit replication. For example, ligation of a short oligoribonucleotide, A₁₀, to the 5′ nucleotide of MDV-1 RNA rendered the RNA unable to replicate exponentially (Miele, Ph.D. thesis, Columbia University, 1982). Attachment of additional nucleotides to the 3′ terminus of other template RNAs similarly inhibits their replication by Qβ replicase. For example, addition of between 10-20 cytidylate residues to the 3′ terminus of Qβ phage RNA abolishes its template activity (Gillis, E., Devos, R. and Seurinck-Opsomer, C., Arch. Int. Physiol. Biochem. 84: 392-393 (1976)); addition of a short oligoadenylate tract has a similar effect (Gilvarg, C. Jockusch, H. and Weissmann, C., Biochem. Biophys. Acta 414, 313-8 (1975); see also Devos, R., van-Emmelo, J., Seurinck-Opsomer, C., Gillis, E., and Fiers, W., Biochim. Biophys. Acta. 447:319-27 (1976)). WO 87/06270 to Chu et al. intimate that attachment of an affinity molecule might be possible to the RNA template provided it is done through a purine linkage so that may be subjected to an acid depurination cleavage procedure prior to replication. Accordingly, the art has shown this technique does not work and thus this approach has not been used as a means to attach probe sequences to the replicatable RNA.

It is yet still another aspect of the present invention to provide new probes and methods for their use which allow for greater flexibility of the placement of the nucleic acid probe sequences in conjunction with the autocatalytic RNA polymerase templates.

It is still yet another aspect of the present invention to provide replicatable RNA templates employing target specific, nucleic acid probe sequences attached to the 5′ terminus of the replicative RNA.

It is a further aspect of the present invention to provide probes and methods for their use involving target specific, nucleic acid probes attached to the 3′ terminus of a Qβ replicative RNA.

Foster and Symons (Cell, 50:9-16 (1987)) and Uhlenbeck (Nature, 328:596-600 (1987)) have described RNA sequences, found in plant virusoids and elsewhere, possessing the unique property of undergoing autocatalytic cleavage in the presence of divalent cations. One such structure, now referred to as the "hammer-head" structure, involves the junction of three double-stranded helices at a region of defined and absolute sequence conservation. Uhlenbeck has shown that the "domains" of this structure may be on separate RNA molecules provided that they combine with each other through hybridization. W. Gerlach (Adeleide, Australia) has used an RNA bearing a portion of such a structure to cleave another totally unrelated RNA bearing the remaining elements, showing that in vitro, the message for chloramphenicol acetyl transferase may be targeted for cleavage by selecting appropriate RNA sequences to form the proper structure.

These "hammer-head" like structures have acquired the name "ribozymes", the name used herein, since one of the two RNAs in the reaction, like a true catalyst, is not consumed during the reaction and can be used to cleave unlimited numbers of the substrate RNAs.

It is a still further aspect of the present invention to provide probes and methods for their use which employ replicatable RNA templates in conjunction with ribozymes for the detection of target polynucleotides.

As will become apparent to those skilled in the art, since as little as a single template RNA molecule can lead to detectable product, any approach in which a hybridization probe is amplified is limited by the background which will be produced by replication of probe molecules carried through the hybridization process irrespective of the presence of target. For example, in a hybridization assay employing a replicatable RNA probe in which target nucleic acid was immobilized on nitrocellulose, it was found that 10⁵ probe molecules were bound, regardless of whether target was present or not (Lizardi et al., supra). Such background limits the sensitivity of such assays since targets present in numbers equal to or below this level will not contribute significantly to the yield of amplified product and thus will not be reliably determined.

It is a yet further aspect of the present invention to provide RNA template probe construct and methods which are less sensitive to background limits.

One approach to reducing this background employs a method by which the target-probe complex is reversibly bound to the support ("reversible target capture"). After hybridization and immobilization, the complex is eluted from the support, which is then discarded with the non-specifically bound probe. The complex is then recaptured on fresh support. This process may be repeated several times to produce a significant reduction (for example, 10⁹-fold) in the amount of non-hybridized probe (see Collins, European Patent Application No. 87309308.2, for a detailed description of this target-cycling technique).

It is further still yet another aspect of the present invention to provide constructs and probes which may be used with such reversible target capture techniques.

In accordance with the principles and objects of the present invention there is provided a probe comprising an RNA template that has a 3' end and a 5' end and is replicable by an RNA-directed RNA polymerase, the probe further comprising first and second nucleic acid probe sequences at, or near, respective ends of the RNA template, the probe adapted so that upon hybridization with a target nucleic acid, a ribozyme is formed, which ribozyme, upon cleavage, releases the RNA template in a replicable form. This probe may have the features of claims 9-14.

This probe can be used in a method of detecting a target nucleic acid having a first and a second nucleic acid sequence in a sample suspected of comprising the target nucleic acid, the method comprising:
(a) where necessary obtaining the target nucleic acid in a single stranded form;
(b) further providing a probe comprising:
   (i) an RNA template having a 3' end and a 5' end, and being replicable by an RNA directed RNA polymerase;
   (ii) a first nucleic acid probe sequence at or near the 5' end of the RNA template and substantially complementary to the first nucleic acid sequence of the target nucleic acid (first target sequence); and
   (iii) a second nucleic acid probe sequence at or near the 3' end of the RNA template and substantially complementary to the second nucleic acid sequence of the target nucleic acid (second target sequence);
(c) contacting the sample with the probe under conditions that allow hybridization to occur between the first and second nucleic acid probe sequences and the first and second target sequences, respectively, to form a ribozyme comprising sequences from the probe;
(d) inducing ribozyme cleavage which releases the RNA template from the target nucleic acid;
(e) allowing the released RNA template to be replicated; and
(f) detecting replicated RNA template as an indication of the presence or absence of the target nucleic acid in the sample.

However, also within the scope of the present invention is a method of detecting a target nucleic acid having a first and second nucleic acid sequence in a sample suspected of comprising the target nucleic acid, the method comprising:
(a) where necessary obtaining the target nucleic acid in single stranded form;
(b) further providing a first probe comprising:
   (i) an RNA template having a 3' end and a 5' end, and being replicable by an RNA directed RNA polymerase; and
   (ii) a first nucleic acid probe sequence at or near one end of the RNA template and substantially complementary to the first nucleic acid sequence of the target nucleic acid (first target sequence);
(c) additionally providing a second probe comprising:
   (i) a second nucleic acid probe sequence substantially complementary to the second nucleic acid sequence of the target nucleic acid (second target sequence); and
   (ii) a third sequence at or near one end of the second probe, and being substantially complementary to a portion of the first probe;
(d) contacting the sample with the first and second probes simultaneously or separately under conditions that allow hybridization to occur between the first and second probe sequences and the first and second target sequences, respectively, and between the first and second probes to form a ribozyme comprising sequences from the first and second nucleic acid probes;
(e) inducing ribozyme cleavage which releases the RNA template from the target nucleic acid;
(f) allowing the released RNA template to be replicated; and
(g) detecting replicated RNA template as an indication of the presence or absence of the target nucleic acid.

These methods may have the steps disclosed in claims 3-7.

Probes for use in the present invention may be provided in a kit which optionally includes instructions for use.

This kit may have the features disclosed in claim 16.

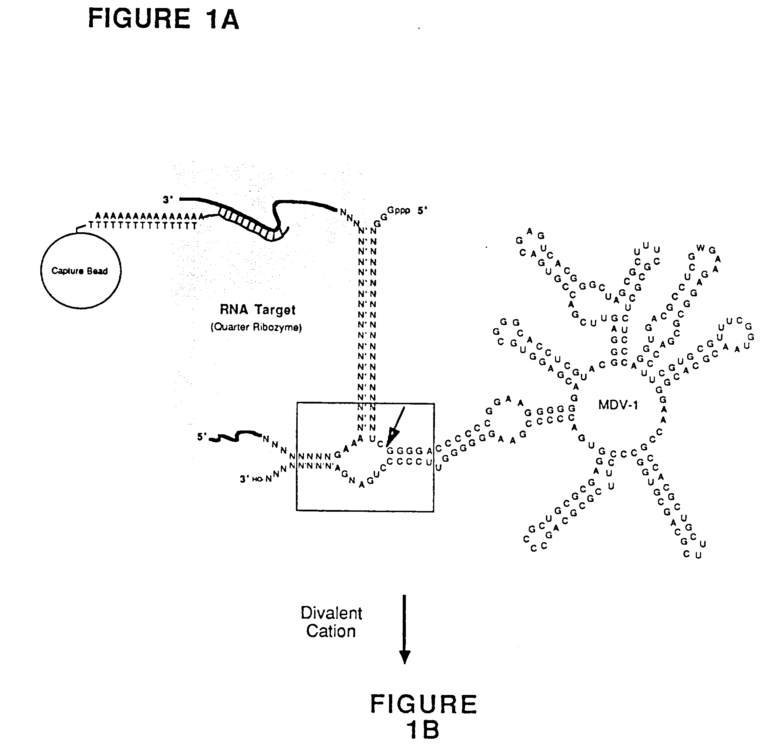

The inhibition produced by a sequence extension on the 5' end of MDV-1 is advantageously employed in the present invention whereby a ternary hybrid formed between a replicatable RNA bearing a 5' probe extension, a second RNA probe, and a target RNA produces an autocatalytic RNA structure (ribozyme) which cleaves the 5' extension from the replicatable RNA probe, simultaneously converting it to an efficiently replicating form and effecting its release from the support. This approach is less preferred since it requires a target RNA containing the specific sequence element 5'-GAAA-3' to generate an active ribozyme structure. The characteristic inability of probe molecules to replicate until specifically bound to target has led to their description as "smart" probes since they provide another approach for reducing background in hybridization assays. The ribozyme structure is the "circular" structure shown in greater detail within the "boxed" portion of Figure 1A. The arrow shows the site where cleavage occurs in the presence of divalent cations such as manganese, magnesium, or calcium.

In another preferred embodiment of the present invention, a nonspecific sequence is placed between the probe and replicatable RNA sequence. The sequence of this "spacer" element may be advantageously altered such that the limits of detection (i.e.-the fewest number of molecules which lead to a detectable product after incubation with Qβ replicase) may be optimally adjusted. A method to generate and select an optimal spacer sequence starting from a population of otherwise identical RNA constructs bearing a defined region of random sequence between the replicatable RNA and the probe sequence elements is provided.

In another most preferred embodiment, a pair of probes are constructed to bind adjacent sites in the target nucleic acid. One of the pair of probes preferably comprises a Qβ RNA template having either a 3' or 5' extension bearing a probe sequence element separated from the replicatable RNA by a spacer sequence. The second probe comprises anoligonucleotidecoupled to a release agent comprising a nuclease which is a ribozyme (or an affinity reagent which in turn can bind the nuclease) which directs endonucleolytic cleavage within the spacer sequence of the first probe. The nuclease is ideally any member of a class of ribozymes which are inactive until a cofactor essential for its activity is added. Both probes may be advantageously employed in a hybridization assay in which the target nucleic acid along with the bound probes are immobilized. After washing away the bulk of the unbound probes, the cofactor required for the activity of the ribozyme is added, causing cleavage and release of the replicatable RNA bearing a short extension into solution. This RNA may then be advantageously amplified by Qβ replicase.

A number of other embodiments of smart probe systems employing such extended Qβ templates are provided.

While the most preferred probes of the present invention will employ target specific RNA sequences at both the 3′ and 5′ termini of the RNA replicatable template, the present invention will also work with DNA probes in these locations, however, it will be readily perceived that there will be greater difficulty in constructing and producing a mixed DNA-RNA probe of the present invention. The sequences required to form the ribozyme, however, must be RNA (and as specified in the ribozyme structure depicted in Figure 1A) in order for the ribozyme structure to form satisfactorily and be subject to inducible cleavage. Such cleavage will, in the most preferred embodiment, occur in the presence of magnesium which also serves as a cofactor for Qβ replicase directed replication of the RNA template, ideally a midivariant. While manganese does work, it is less preferred since template specificity may be altered and, therefore, replication may not occur with the desired fidelity.

The most preferred RNA template is MDV-1 or midivariant, a 221 nucleotide RNA, described by Chu et al., WO 87/06270, and found in cells infected with bacteriophage Qβ. MDV-1 is a common contaminant of Qβ replicase preparations. Qβ replicase acts upon (i.e. replicates) RNA templates which have particular primary and secondary structures such as MDV-1 and other structures. MDV-1, while replicated along with the Qβ RNA genome, appears to have no known function. It is also known to exhibit some natural variation in the 5′ and 3′ termini, generally simply seen as an addition or deletion of a GC pair, shown by way of explanation with 4 GC pairs in Figures 1 and 2 and with 3 GC pairs in Figures 3 and 4.

The more preferred probe embodiments of the present invention will employ a relatively "short" target specific probe sequence at the 3′ terminus of the RNA template. Most preferably, this target specific sequence will have a length of about 4 to about 12 bases and ideally, a length of about 5 bases. Such a relatively short length is preferred because following cleavage of the ribozyme those will favor disassociation of the replicatable RNA template from the target sequence at a typical cleavage temperature of about 55°C.

It will of course be readily recognized by those skilled in the art that increasing the length of the target specific probe sequence on the 5′ terminus of the replicatable RNA template will generally foster increasing target specificity. It is also most advantageous that such a probe have a sufficient length such that attempted replication of the template with this 5′terminus target specific probe still attached will result in template collapse prohibiting further replication. On the other hand, if both the 5′ and 3′ target specific probes are short probes, e.g. only 5 nucleotides in length, then in this embodiment one has the possibility of constant formation and turnover of ribozymes resulting in a yet more rapid cleavage of replicatable RNA templates. The disadvantage of this embodiment compared to the most preferred embodiment employing a 5′terminus target specific sequence of substantially greater length is that one would expect some loss in target specificity.

The most preferred probe of the present invention will comprise a midivariant as the RNA template having a 3′ end and a 5′ end and having a 5′ end leader extension comprising a target specific first nucleic acid probe sequence complementary to a first target nucleic acid sequence followed by 5′-UC-3′ and further having a 3′ end trailer extension comprising 5′-CUGANGA-3′ followed by a second nucleic acid probe sequence complementary to a second target nucleic acid sequence and wherein the midivariant most preferably has the sequence shown in Figure 1A beginning at the 5′ end with GGGGACC... and ending at the 3′ end with ...GUUCCCC. Alternatively in another preferred embodiment, the midivariant will have one of the four C's on the 3′ end shown in Figure 1A deleted allowing the U (on the 3′ end and just inside the box shown in Figure 1A) to pair with a G thereby allowing the stem to "straighten" thus eliminating the possibility of isomerization in such stem which might otherwise result in a mispairing along the remainder of the stem. Such a mispairing would ultimately lead to misformation and thus inactivity of the ribozyme, an event advantageously avoided.

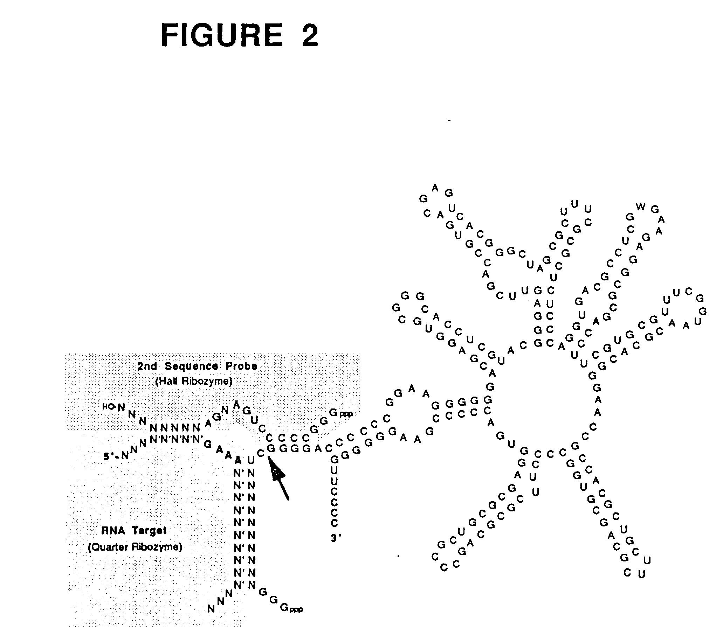

An alternative preferred embodiment (shown in Figure 2) utilizes a probe construction wherein the first nucleic acid probe sequence specific for the RNA target is appended to the 5′leader sequence of the replicatable RNA template as in the preferred embodiment. Unlike the most preferred embodiment, however, the 3′ terminus of the replicable RNA template does not incorporate a target specific nucleic acid probe sequence. Rather, a second probe comprising a second nucleic acid probe sequence specific for a second RNA target sequence, half of the ribozyme structure, and a third nucleic acid probe sequence specific for a portion of the 5′ RNA template stem sequence is utilized. As with the most preferred embodiment, hybridization of both probes with the RNA target results in the formation of a cleavable ribozyme in which the RNA target provides a requisite 5′-GAAA-3′ sequence.

The most preferred methods of the present invention comprise contacting the preferred probe constructions of the present invention with single-stranded target nucleic acid under conditions allowing hybridization of the first and second nucleic acid probe sequences with the first and second target sequences (having an intervening sequence of 5′-GAAA-3′respectively whereby a ribozyme is formed. That ribozyme is then advantageously cleaved, for example by bringing into contact therewith a divalent cation selected from the group of manganese, magnesium and calcium. The RNA template, ideally the MDV-1 midivariant, is then advantageously released from the target nucleic acid because the "short" second nucleic acid probe sequence at the 3' end of the RNA template will be of insufficient length to stably hybridize to the RNA target. The released RNA template may then be advantageously replicated by Qβ replicase and the replicated RNA template detected as an indication of the presence or absence of the target nucleic acid. The most ideal methods will include a separation step prior to cleavage induction whereby non-hybridized probe is removed and therefore unable to participate as an available template in the amplification cycle by Qβ replicase. Removal may be accomplished by a variety of means such as for example by immobilizing the probe - target hybridization complex via another insolubilized target specific probe, itself directly or indirectly immobilized upon a solid surface such as a capture bead, dipstick, etc. Such so called "sandwich" techniques are well known by those skilled in the art and need not be explored in detail here.

### Brief Description of the Drawings

Further understanding of the principles, objects and other aspects of the invention may be had by study of the accompanying drawings wherein:
Figure 1A illustrates the most preferred embodiment showing hybridization of a midivariant with an RNA target forming a ribozyme (the "circular" structure contained within the box) showing the cleavage site;

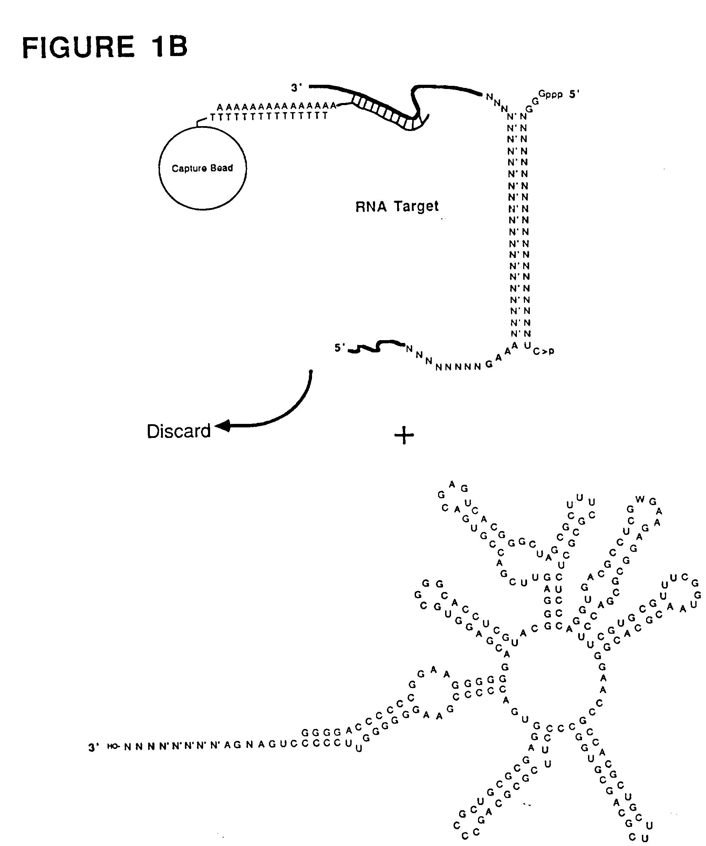

Figure 1B illustrates the resultant cleavage products obtained by the addition of divalent cation to the probe-target complex of Figure 1A;
Figure 2 illustrates an alternative embodiment wherein a first probe comprising an RNA template and a 5' terminus target specific sequence hybridizes to the RNA target and wherein a second probe sequence hybridizes to both the RNA target and a 5'stem sequence of the RNA template of the first probe whereby a ribozyme is formed;

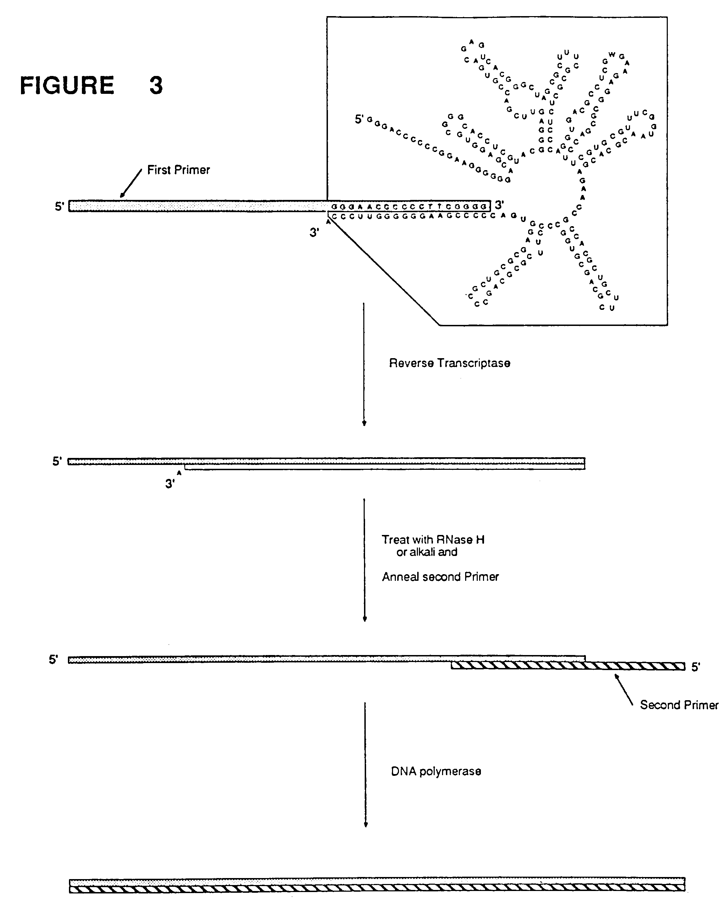

Figure 3 illustrates graphically the general construction scheme of a cDNA clone for use in generating probes of the instant invention;

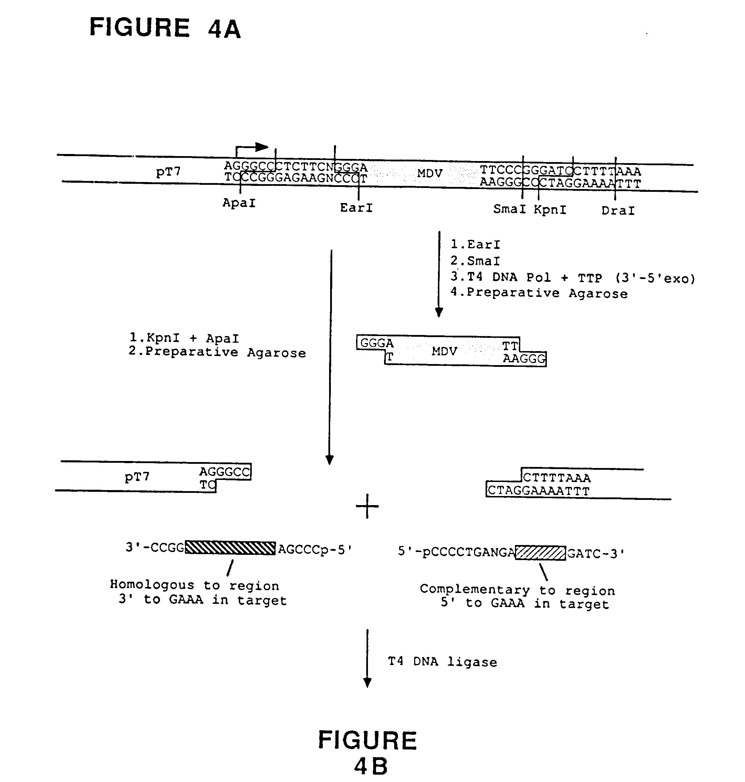

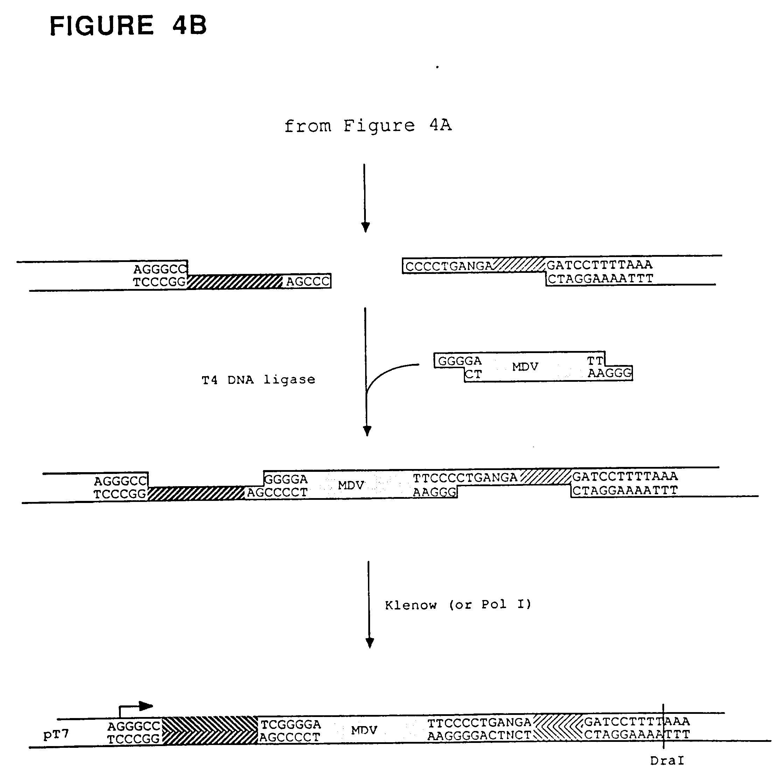

Figures 4A and 4B illustrate graphically the use of the cDNA clone produced in Figure 3 in one construction of a vector to produce the most preferred probe of the instant invention; and

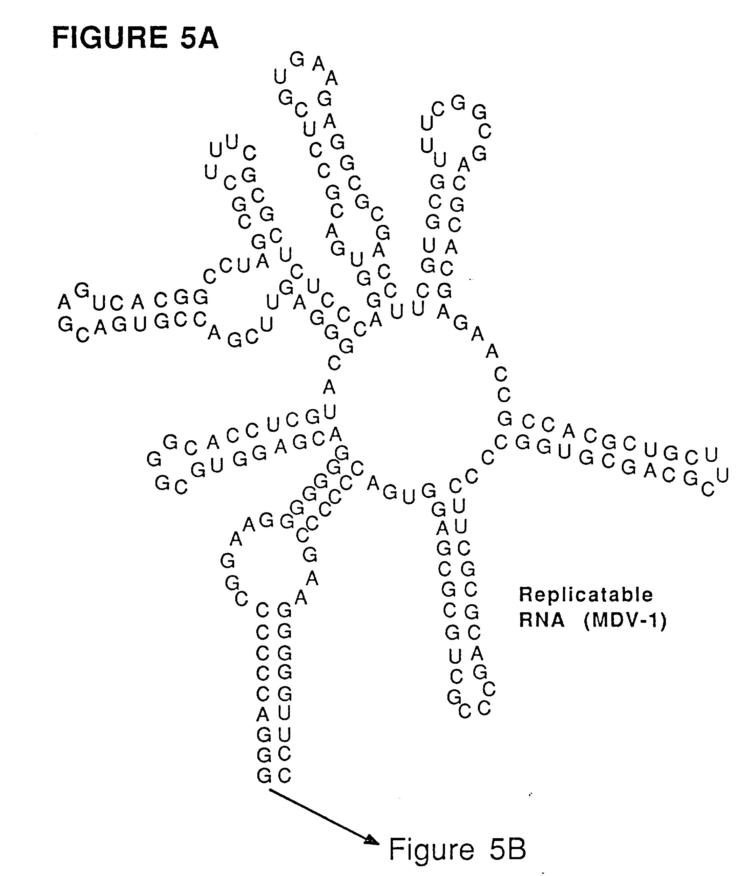

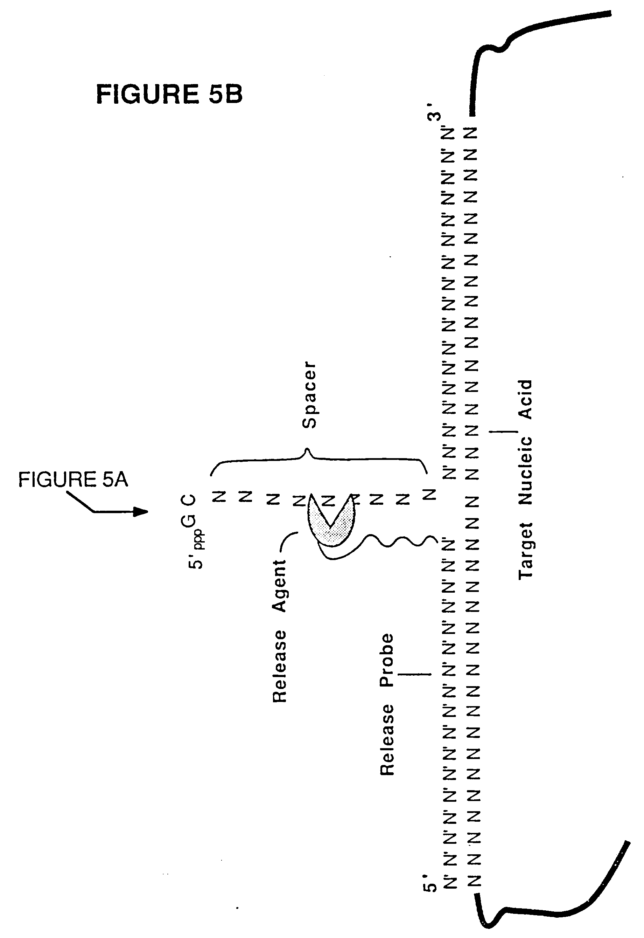

Figures 5A and 5B graphically depict the most preferred embodiment employing a pair of probes, one of which comprises the midivariant-1 attached to a target specific probe through a cleavable spacer element.

In an examination of the results of Miele (supra) and others, a set of recombinant MDV-1 RNAs bearing 3′ terminal extensions of various lengths was generated by transcription of a cDNA clone described by Lizardi et al. (supra) which had been digested variously with restriction enzymes whose cleavage sites occur downstream of the MDV cDNA. It was surprisingly discovered that, contrary to the general teachings of the literature, a 3′ appendage as long as 183 nucleotides in length to the 3′ terminus of the recombinant RNA template allowed sufficient replication to occur such that amplified RNA products could be detected when as few as 10⁴ molecules bearing such 3′ extensions were used to initiate a Qβ replicase reaction. The amplified product RNAs were identical in size to the RNA lacking a 3′ extension, indicating the 3′ extension was not replicated with the template portion of the molecule. This surprising and totally unexpected result has significant implications for the application of replicatable RNAs as probes in amplified hybridization assays since it provides for greater flexibility in the placement of probe sequences on the replicatable RNA.

### Example 1: Production of a Ribozyme: Qβ template RNA probe Chimera.

The RNA construct illustrated in Figure 1 is produced by the following method. Two synthetic oligonucleotide primers are produced by methods familiar to those skilled in the art. The first contains one strand of a number of recognition sequences of the restriction endonucleases used for subsequent constructions (see Figure 4A), joined to a portion complementary to the sequence proximal to but not containing the ultimate 3′ nucleotide, A, of the plus strand of midivariant RNA, a template for the enzyme Qβ replicase. As will be understood by those skilled in the art, the exact length of a sequence complementary to this region of the RNA must be of sufficient length to form an intermolecular hybrid and displace the intramolecular base pairing present in the midivariant RNA in this region. The second oligonucleotide contains, in order from its 5′ to 3′ end, the sequence comprising one strand of the promoter for a DNA-dependent RNA polymerase read in the same sense as the direction of transcription. In this case, said promoter is that for the RNA polymerase encoded by the bacteriophage T7, a highly efficient enzyme, although as will be understood by those skilled in the art, promoters for other RNA polymerases can be used as well. This sequence element is followed, in the 3′ direction by a cleavage site for a restriction endonuclease which produces a staggered break in double-stranded DNA, and which leaves a protruding single-stranded 3′ terminal extension. In this example, this enzyme is Apa I, and its sequence is made to partially overlap the promoter for T7 RNA polymerase. This site is followed by a recognition sequence for a restriction endonuclease which may be any member of a class of enzymes which recognize a sequence and cleave the DNA in a region 3′ to that sequence element, and which leave a staggered cleavage and a protruding single-stranded 5′ terminal extension. This recognition sequence is placed in such a manner as to direct the cleavage of the final DNA vector construct in one strand immediately prior to the sequence encoding the midivariant RNA. In this example (see Figure 4A), this is the site for the restriction endonuclease Ear I which recognizes the sequence CTCTTCN, where N is any nucleotide, and cleaves the DNA in the same strand after the nucleotide N, and three nucleotides further in the complementary strand. Finally, this sequence is followed by another sequence homologous to the sequence proximal to and including the 5′ terminus of midivariant RNA (MDV-1 RNA) which is of sufficient length to anneal and prime the synthesis of a second strand product as described below, except that deoxyribonucleotides replace the ribonucleotides found in MDV-1.

The first oligonucleotide is used as a primer for the synthesis of a cDNA of midivariant RNA as shown in Figure 3. MDV-1 is obtained from a Qβ replicase reaction initiated without exogenous template RNA. This will generate abundant levels of MDV-1 RNA because all preparations of Qβ replicase holoenzyme prepared by any of a number of methods typically are contaminated by low levels of this RNA. One picomole each of the first oligonucleotide described above and MDV-1 RNA are mixed and annealed by heating and allowing the mixture to cool slowly. This primer-template combination is used to initiate a reverse transcriptase reaction in a manner such as those described by Maniatis et al., A Cloning Manual. After completion of the first strand synthesis, the midivariant RNA is destroyed by mild alkali treatment or alternately, RNase H. The second complementary strand is generated by annealing the product of this reaction with the second oligonucleotide described above and extending with a DNA-dependent DNA polymerase. This product can then be cloned into a selectable, replicatable DNA vector in a manner such as that described by Maniatis et al. Potential clones are screened for the presence of each of the restriction endonuclease sites included in the oligonucleotides by conventional methods.

After propagation in and purification from bacteria, the cloned cDNA is cleaved with the restriction endonuclease Apa I and Kpn I. The smaller fragment is purified electrophoretically, and cleaved with the restriction endonuclease Hinf I. This enzyme cleaves the cDNA at the site corresponding to position 65 in MDV-1. In the naturally occurring population of MDV-1 RNAs, however, this site occurs in only a fraction of the molecules. Thus, a number of clones must be screened for those which possess the appropriate sequence. Once thusly obtained, the Hinf subfragments are ligated to the oligonucleotide 5′-pGA₁₀-3′ annealed to the oligonucleotide 5′-pCT₁₀-3′. This ligation mixture is digested with Apa I and Kpn I. The resultant mixture is ligated into the large fragment resulting from digestion of the vector produced above with Apa I and Kpn I. This will generate a clone in which the DNA sequence encoding the midivariant is interrupted by tract of A residues. Any of a number of sequences may be substituted for the A₁₀ tract utilized in this example. However, A₁₀ is known to be maintained during replication of midivariant RNA, as shown by Miele et al. (J. Mol. Biol. (1983) 281-295).

After propagation in and purification from bacteria, the cloned DNA from the construction above is cleaved in two separate reactions (see Figure 4A). In the first of these, the segment bearing the complete midivariant RNA cDNA is excised with the enzymes Ear I and Sma I. This digest is treated with T4 DNA polymerase in the presence of thymidine triphosphate to remove three nucleotides from the 3′ end of the strand bearing the sequence in the same sense as the MDV-1 plus strand (e.g. the strand shown in Figure 1, its complement is the minus strand, also a replicatable RNA template). The resulting modified fragment is purified by electrophoresis through polyacrylamide gels. The second restriction digest is with the enzymes Apa I and Kpn I. The larger of the two fragments is purified by agarose gel electrophoresis.

Two oligonucleotides are synthesized. The first of these advantageously contains the sequence 5′-CCCCTGANGA-3′ followed by at least four nucleotides complementary to the sequence in the target RNA 5′ to the 5′-GAAA-3′ element and terminating in the sequence 5′-GATC-3′. The second oligonucleotide ideally contains the sequence 5′-CCCGA-3′ followed by at least 4 nucleotides of the sequence 3′ to the 5′-GAAA-3′ element in the target, except that deoxyribonucleotides replace the ribonucleotides of the target. This element is advantageously followed immediately by the element 5′-GGGG-3′. Each of the oligonucleotides is phosphorylated on its 5′ terminus by T4 polyndcleotide kinase.

Both of the oligonucleotides are ligated to the large fragment produced by the Kpn I, Apa I digest of vector DNA in a reaction containing T4 DNA ligase and ATP such as those described by Maniatis et al (ibid). The ligation product (see Figure 4B) is purified away from unligated oligonucleotides by gel filtration. The ligation product is then ligated to the small Ear I, Sma I fragment bearing the modified terminus. The resulting "gapped" molecule is rendered fully double-stranded by treatment with a DNA-dependent, DNA polymerase. This product may be advantageously introduced into and propagated in bacteria.

As will be obvious to those skilled in the art, this series of manipulations will generate DNA clones in which the MDV cDNA portion is inserted in both its normal and inverted orientation relative to the direction of transcription by T7 RNA polymerase. The clones containing the MDV cDNA in plus strand orientation (oriented such that transcription produces an RNA containing the plus strand of MDV-1) are determined by screening for restriction endonuclease fragments of the appropriate size which are known to cleave within the MDV-1 cDNA sequence.

The DNA prepared from a clone with the MDV-1 in the plus strand orientation is cleaved at a restriction site distal to the second probe element relative to the promoter. In this example, this is the restriction endonuclease Dra I. This digested DNA is advantageously transcribed by T7 RNA polymerase in vitro under conditions such as those described by Milligan et al. (Nucl. Acid. Res. (1987) 15: 8783-8798) to generate the RNA probe.

### Example 2: Assembly of a Bimolecular Ribozyme Probe Set

The cDNA clone as described in Example 1 is cleaved with the enzymes Apa I and Ear I, and the large fragment is ideally purified away from the small fragment described above. An oligonucleotide having the sequence 5′-CCCGA-3′ followed by 4-50 nucleotides identical to the sequence immediately 3′ to a 5′-GAAA-3′ element in the target, in turn followed by the sequence 5′-GGCC-3′ is synthesized by any of a number of methods familiar to those skilled in the art. This oligonucleotide is annealed and ligated to the large restriction fragment, and the resulting single stranded "gap" region rendered double-stranded by the action of a DNA-dependent DNA polymerase. This DNA is introduced into and propagated within bacteria. After purification from bacteria, it may be cleaved with the restriction endonuclease Sma I, and then used as a template in an in vitro transcription reaction utilizing bacteriophage T7 RNA polymerase.

The second sequence probe may be generated by constructing a synthetic DNA template for T7 RNA polymerase as described by Milligan et al. (Nucl. Acid. Res. (1987)15:8783-8798), one strand of this template starting at its 5′ end with at least 4 nucleotides of the sequence from the region 5′ to the 5′-GAAA-3′element in the target except that deoxyribonucleotides replace the ribonucleotides found in the target, followed by the sequence 5′-TCNTCAGGGGGCCCTATAGTGAGTCGTATTA-3′ where N indicates any nucleotide. The other strand of the template is an oligonucleotide having the sequence 5′-TAATACGACTCACTATAG-3′. These two oligonucleotides preferably are mixed and transcribed in vitro as described by Milligan et al. (ibid). The product may be readily purified by any of a number of methods familiar to those acquainted with the art.

### Example 3: Replication of modified MDV-1 RNAs bearing 3′-terminal extensions.

A cDNA clone of MDV-1 similar to that described in example 1, was obtained from F.R. Kramer of Public Health Research Institute, New York City, and contained a promoter for T7 RNA polymerase, and a modified MDV-1 cDNA sequence in which the sequence element 5'- CTCTAGATCTCGAGA CTAACATAGGTCTTAACTTGACTAACATCGAGGCCTGCTAGAG-3′ replaced the 3-nucleotide segment encoding nucleotides 64-66 in the naturally-occurring MDV-1 RNA, followed by the sequence 5'-GGGAATTC-3'. This entire sequence was cloned between the Hind III site and the EcoRI site of pSP64 (Melton et al., Nucl. Acid. Res., Vol. 12, 7035-7056). This plasmid was cleaved separately with the restriction endonucleases Sma I, EcoRI, Alu I, and Pvu II, and each of the cleaved preparations transcribed in vitro by T7 RNA polymerase under conditions described by Milligan et al. The RNAs produced in these reactions were purified by denaturing polyacrylamide gel electrophoresis. This procedure resulted in the generation of modified MDV-1 molecules bearing either no extension beyond the 3' terminus found in the naturally-occurring MDV-1 RNA, a 7-nucleotide 3' extension, a 24-nucleotide 3' extension, or a 183-nucleotide 3' extension.

The purified RNAs were serially diluted and 10⁴ molecules used to prime a Qβ replicase reaction as described by Chu et al. (Nucl. Acid. Res. (1986) 14:5591-603), except that the Qβ replicase was that purified from the expression clone of the replicase described by Biebricher et al. (Nature (1986) 321:89-91) and physically obtained from C. Biebricher (Max Plank Institute). After 30 minutes, two microliters of each reaction was mixed with 18 microliters 95% formamide, heated to 100°C for 5 minutes, and resolved by electrophoresis on an 8% polyacrylamide gel containing 8.3M urea. The replicated recombinant RNAs were distinguished from the MDV-1 RNA which contaminates the enzyme preparation and is also replicated, by their larger size resulting from the larger sequence replacing nucleotides 64-66, which in turn results in a lower electrophoretic mobility. The recombinant RNA product represented approximately 50%, 86%, 72%, and 15% of the total RNA when the reaction was initiated with the modified MDV-1 RNAs bearing either no 3′ extension, the 7-nucleotide extension, the 24-nucleotide extension, or the 183-nucleotide extension, respectively. The recombinant RNAs produced in each case were the same size as the recombinant RNA which lacked a 3′ terminal extension, indicating that the 3′ extension itself is not replicated with the rest of the molecule. This experiment has been repeated with a number of 3′ terminal extensions of different sequence with comparable results.

### Example 4: An assay for target nucleic acid utilizing a ribozyme probe.

Two deoxyribo-oligonucleotides are synthesized with the following sequence: 5′ CCCGAGGATCACCAGCAATATTCCAAAGTAGCATGACAAAAATCTTGGCC-3′ and 5′-CCCCCTTGACGACATCCCGATC-3′. These are cloned into the cDNA vector described in Example 1, and the purified cloned DNA transcribed with T7 RNA polymerase after cleavage with the restriction endonuclease Dra I. The RNA product of the correct size is purified by denaturing polyacrylamide gel electrophoresis.

One picomole (10⁻¹ mol) of this RNA is hybridized to whole human blood from patients infected with human immunodeficiency virus type 1 (HIV-1), normal human blood, or a sample to be assayed for the presence of HIV-1, in a sandwich hybridization assay such as described by Ranki et al. (ibid) in the presence of 2.5M guanidine thiocyanate and one picomole of a synthetic deoxyribo-oligonucleotide having the sequence 5′- GGAAGCACATTGTACTGATATCTAATCCCTGGTGGTCTCATA₁₅₀-3′. With general reference to Figure 1A the hybrid is bound to a solid support by hybridization of the dA150 tract in the oligonucleotide to immobilized polydeoxythymidine such as in the well of a polystyrene microtiter plate (or a capture bead as shown in Figure 1A) coated with dT₃₀₀₀ according to the methods of Collins et al., (EPA 87309308.2 and EPA 88312135.2). The plate is incubated at 37°C for 30 minutes after which the contents, containing unbound probes, are aspirated and discarded, and the well repeatedly washed with a buffer containing 0.5M guanidine thiocyanate. The well is then washed with a buffer containing 90mM Tris HCl (pH 7.5), and 50µl of the same buffer except containing 14mM MgCl₂ introduced into the well and incubated at 50°C for 15 minutes to induce ribozyme cleavage. The contents of the well containing the cleaved and released probe (see Figure 1B) are transferred to another well for replication. Five microliters of a solution containing 4mM each ATP, GTP, CTP, and UTP, 10µCi of α-³P-CTP and 1ug of Qβ replicase are added and the plate incubated at 37°C for 25 minutes. Two microliters of the well contents are removed and added to 18µl of 95% formamide, 0.05% xylene cyanol, 0.05% bromphenol blue for halting replication. Five microliters of this are applied to the well of a denaturing 8% polyacrylamide gel, and the replicated RNAs resolved by electrophoresis until the xylene cyanol dye has migrated the length of the gel. An X-ray film is exposed to the gel for three hours and is then developed as the final step of detection. The larger RNA product of length equal to the number of nucleotides between the sequence corresponding to the unappended 5′ terminus of the naturally-occurring MDV-1 RNA and the unappended 3′ terminus of MDV-1 RNA and including the length of the sequence element inserted into the Hinf site such as described in Example 1 (in that case 10 nucleotides), indicates the presence of HIV-1 virus or its messenger RNA in the blood sample. It will also be readily recognized that by carefully timing the replication phase and comparing the results against a suitable control, correlation can be made between the amount of replicated RNA with the amount of target RNA in the original sample thereby providing quantitative results.

As will be readily understood, other methods of detecting the replicated RNA may be employed including immunological methods utilizing antibodies specific for RNA-DNA hybrids (e.g. requires addition of DNA oligonucleotides specific for replicated RNA sequence) or alternatively, using ethidium bromide, the fluorescence of which is enhanced by the presence of RNA polymers.

### Example 5: A pair of probes against HIV-1, the causative agent of human acquired immune deficiency syndrome, were generated. Two pairs of partially complementary oligonucleotides were generated with the sequences:

(1A) 5′-GTGTGTGTGTAAGATGTTCAGCCTGATCTCTTACCTGTCCTATAATTTTCG-3′;

(1B) 5'-AATTCGAAAATTATAGGACAGGTAAGAGATCAGGCTGAACATCTTACACACACAC-3';

(2A) 5′-GGGCGGTCGCGCGAAAAAGATGTTCAGCCTGATCTCTTACCTGTCCTATAATTTTCG-3′;

and

(2B) 5′-AATTCGAAAATTATAGGACAGGTAAGAGATCAGGCTGAACATCTTTTTCGCGCGACCGCCC-3′.

Each of these oligonucleotide pairs (1A and 1B; 2A and 2B) were annealed and separately cloned between the EcoRI site and the SmaI site of the plasmid used in the above experiment. After cloning, the purified plasmid DNAs were digested with EcoRI and transcribed with T7 RNA polymerase to generate two RNAs bearing 3' extensions which were in part complementary to the same sequence in HIV-1. In the RNA produced by the clone obtained with the first pair of oligonucleotides, the sequence of the replicatable RNA and the probe sequence was separated by the spacer sequence element: 5'-GUGUGUGUGU-3'. In the second clone, a spacer of the sequence 5'-GGGCGGUCGCGCGAAA-3' was generated. Each of the RNAs was serially diluted and aliquots used to initiate Qβ replicase reactions containing 1µg of Qβ replicase, 90mM Tris HCl, 14mM MgCl₂ and 400µM each ATP, GTP, CTP and UTP. After 30 minutes at 37°C, the reactions were stopped by addition of EDTA to 20mM and ethidium bromide to 1µg/ml. Fluorescence of the ethidium bromide-RNA complexes was observed over an ultraviolet transilluminator. Amplified product RNA was observed in reactions initiated with at least 10⁴ molecules generated by the first clone, and 10 molecules generated by the second clone. These results indicate the sequence region immediately adjacent to the 3′ end of the replicatable RNA sequence (i.e.-the spacer) strongly affects the sensitivity of probe detection. Subsequent experiments indicated that increasing the reaction time did not affect the ultimate dilution yielding a detectable product by fluorescence.

These results provide practical guidance for using replicatable RNAs bearing 3′ sequence extensions as probes for the sensitive detection of nucleic acids. By appropriate selection of the spacer sequence, RNA probes may be advantageously generated which have intrinsically greater sensitivity in the detection of target nucleic acid. Conversely, for some target nucleic acids which are present in an infectious agent at high levels (i.e.- ribosomal RNA, present at up to 50,000 copies per organism), a spacer region conferring relatively poor limits of detection may be utilized to advantageously avoid the background otherwise generated by the amplification of lower levels of non-specifically bound probes. For example, if reduced in number to levels at least an order of magnitude below the limit of detection using background reduction methods such as Collins (supra), non-specifically bound probes will be incapable of producing a detectable signal. Thus, the probes of the present invention comprising appropriate spacer sequences may be used advantageously to reduce the cost, complexity, and frequency of false-positive reactions in such assays.

As will now be recognized, a number of means may be utilized to generate such constructs including the use of T4 RNA ligase (Stefano, supra) and transcription of cDNA clones as described in the above example. It will be appreciated that the use of T4 RNA ligase advantageously allows the generation of constructs in which the probe extension is either RNA or DNA.

Since Qβ replicase initiates synthesis of the daughter strand at the 3' end of MDV-1 RNA template and generally continues to copy the template in a 3' to 5' direction (of the template) until the 5' terminus is reached, template molecules bearing 5' extensions will generate daughter strand products (e.g. a minus strand of template) bearing 3' terminal extensions, which in turn will be replicated as observed above (e.g. the Q-beta replicase will ignore the 3' sequences appended to the end). As will be apparent to those skilled in the art, such probes may also be employed in hybridization assays. As will now be additionally apparent, the effect of the spacer sequence may similarly be employed to advantageously adjust the sensitivity of such probes.

An important advantage issues from the present discovery which has additional application for smart probe systems which employ the target-directed cleavage of 5' or 3' sequence extensions, since such methods need not provide for the precise and complete removal of those sequences. For example and as set forth previously, a probe may be generated bearing both 3' and 5' extensions whose hybridization to target produces a ribozyme structure which directs cleavage of the 5' extension from the RNA. A short 3' extension remains on the molecule.

A most preferred embodiment of the present invention is diagrammed in Figures 5A and 5B wherein a probe molecule bearing an extension is hybridized to a target in solution. Shortly before, after, or simultaneously therewith, a second oligonucleotide probe is hybridized to the target adjacent to the first probe. The second oligonucleotide probe is coupled to a nuclease which is a ribozyme and which requires a cofactor (for example, a divalent cation) for its activity. Following hybridization, the complex is captured on a solid support in a manner such as that described by Ranki et al. (supra), Soderlund (supra), Stabinsky (supra), and/or Syvanenen (supra). After separating, such as by washing away, the bulk of non-specifically bound probes, the cofactor for ribozyme activity is added. Theribozyme, coupled to the terminus of the probe proximal.to the first probe when hybridized to its target, cleaves the spacer on the first probe, thereby releasing a replicatable moiety into solution. As previously indicated, the spacer sequence may be advantageously selected to maximally inhibit replication until cleavage occurs. Other variations of this approach will become apparent. For example, such probe pairs may include a replicatable RNA instead bearing a 5' extension and a nuclease-coupled probe in which a ribozyme sequence is present 5' to the probe sequence element.

Any of a number of ribozymes may be advantageously employed as the cleavage agent, such as those described by Haselhoff & Gerlach (Nature 334: 585-591 (1988)), Uhlenbeck (1987) Nature 328, 596-600, Cech (European Pat. Appl. WO 88/04300, June 16, 1988), Sharmeen et al. (1989) J. Virol. 63, 1428-1430; or Hampel & Tritz (1988) J. Cell. Biochem., Suppl. 12D, Abst. #N212, p.31, is employed. Ribozymes possess a significant advantage in that the second probe bearing the ribozyme may be efficiently produced in a single step by transcription of a DNA oligonucleotide of appropriate sequence such as is described by Mulligan (supra), thus reducing the cost and labor required to generate such reagents. Moreover, these preferred reagents advantageously produce specific cleavage events, leading to a product RNA with defined replication properties.

Further understanding may be had by study of the additional detailed examples which follow.

### Example 6: Detection of Chlamydia trachomatis RNA

Two oligonucleotides having the sequences: (1) 5′-AATTCTATGTGATATCAGCTAGTTGGTGGGGTAAAGGCCT-3′; and (2) 5′-AATTAGGCCTTTACCCCACCAACTAGCTGATATCACATAG-3′ were annealed and ligated into a MDV cDNA construct similar to that described by Lizardi (supra), which had been digested with EcoRI. This cDNA clone differed from those described by Lizardi et al. in that the internal insert encoded a binding site for the coat protein for phage R17. This cDNA was obtained from F.R Kramer, Public Health Research Institute, N.Y. The oligonucleotides were ligated in the orientation such that upon subsequent digestion with EcoRI, cleavage occurred downstream of the oligonucleotide with respect to the promoter for T7 RNA polymerase. The digested plasmid was transcribed in vitro with T7 RNA polymerase under the conditions described by Mulligan et al. (supra), and the transcription product of correct length isolated by electrophoresis through polyacrylamide gels containing 8.3M urea.

A capture oligonucleotide having the sequence: 5'-TACACCGCTATAAACCCGTAGGCTCATTGCAATTTC-3' complementary to a region in the 16S ribosomal RNA of Chlamydia (Palmer et al., Chlamydial Infections (Oriel et al., eds) Cambridge Univ. Press, 1986, pp. 89-92) was synthesized and tailed with ca 150 dA residues using terminal deoxynucleotidyl transferase (Supertechs) as described by Collins (supra).

Various numbers of formalin-fixed elementary bodies of Chlamydia trachomatis were lysed in a solution of 1.0mg/ml proteinase K (Boehringer Mannheim) and 1.6% Sarkosine (Sigma) at 65°C for 15 minutes in a final volume of 35µl. Thirty-five µl of a buffer containing 340 ng/ml of tailed capture oligonucleotide and 100 ng/ml of the transcription product probe was then added and solution phase hybridization allowed to occur for 30 minutes at 37°C. Fifty µl of a 0.06%(w/v) suspension of oligo-dT derivitized magnetic beads prepared according to Collins (supra) in 4% BSA, 10mH EDTA, 0.2% Sarkosine, 0.1M Tris-HCl pH8.0 and 0.05% bronopol was then added and incubated for an additional 5 minutes at 37°C to capture the target-probe hybrids on the beads.

Following capture, 0.2ml of a "wash solution" containing 1M guanidine thiocyanate (GuSCN, Fluka), 10mM EDTA, 0.04 M Tris-HCl pH7.8, 0.5% Sarkosine, 0.2% BSA and 0.1% antifoam (Thomas) at 37°C was added. The tubes were vortexed and then placed in a magnetic field (preferably such as that described in EPC 88310810.2 entitled "Magnetic Separation Device and Methods for Use",). The beads were drawn to the side of the tubes and the liquid phase removed from the tubes by aspiration. Another 0.2ml of warm wash solution was added, the beads were resuspended by vortexing, and the beads recollected in the magnetic field. The beads were washed an additional time with another aliquot of wash solution.

The collected beads, freed of supernatant, were then resuspended in 50µl of a buffer containing 3.25M GuSCN, 65mM EDTA, 0.04M Tris-HCl, pH7.0, 0.5% Sarkosine, and 0.5% BSA, and incubated at 37°C for 5 minutes to release the target-MDV probe-capture probe hybrids. The magnetic beads were collected as before, and the supernatants removed and transferred to a fresh set of tubes, each containing 50µl of a fresh bead suspension to recapture the hybrids as described above. These beads were washed three times in the same manner as the first set, the hybrids released and recaptured by a third set of beads. This set of beads was washed three times in the same manner, and additionally, three times with 0.2ml of a solution containing 0.1M KCl, 1mM EDTA, 10mM Tris, PH8.0, 0.5% NP-40.

The collected, washed beads were resuspended in 50µl of a buffer consisting of 10mM Tris-HCl, PH8.0, 1mM EDTA, and 0.5% NP-40 and incubated at 37°C for 5 minutes to elute the complexes. The beads were collected with a magnetic field, and the supernatants containing the eluted complexes transferred to a fresh set of tubes.

Ten µl of each of the bead elutes was added to a set of tubes containing 13µl of a solution of 173mM Tris-HCl, pH7.5, 27mM MgCl₂, and 0.77mM each ATP, GTP, CTP, and UTP (Pharmacia). Two µl of Qβ replicase (1.2µg) purified according to DiFrancesco (supra) was added, and the tubes incubated for 12 minutes at 37°C. Five µl of 20mM EDTA, 32µg/ml propidium iodide (Sigma) was then added to stop replication. Fifty µl of each stopped reaction was transferred to separate wells of a U-bottomed microtiter plate (Nunc). The plate was photographed over an ultraviolet transilluminator whose emission maximum was 365nm onto Polaroid® type 667 film using a Wratten® #29 filter. The exposure was adjusted to minimize the apparent fluorescence in a control well containing reaction buffer and propidium iodide alone. The above assay was performed with triplicate samples of each elementary body dilution.

The results are summarized in the table below. The number of "+" symbols reflects the relative fluorescence observed. "-" indicates that the fluorescence did not exceed that of the control wells.

| | Fluorescence | | |
|---|---|---|---|
| Elementary bodies | Hybridization Reaction: | | |
| | a | b | c |
| 10⁵ | +++++ | ++++++ | +++++ |
| 10⁴ | ++++ | ++++ | +++++ |
| 10³ | +++ | +++ | + |
| 10 | ++ | ++++ | - |
| 10 | - | - | - |
| 1 | - | - | - |
| 0 | - | - | - |

As observed in the above experiment, the assay had a sensitivity of approximately 10³ elementary bodies.

### Example 7: Detection of HIV-1 mRNA

The assay of Example 6 was repeated, except that the capture probe was substituted with three probes complementary to conserved regions in HIV-1 RNA. The detector MDV probe was as described in Example 5 and contained the spacer sequence 5′-GGGCGGUCGCGCGAAA-3′. This assay showed a sensitivity of 1000 HIV-1 RNA molecules.

### Example 8: Selection of probes containing optimally-replicating spacer sequences

A library of template DNAs can be generated by synthesis of an oligonucleotide having in 5′ to 3′ order: (1) a short (for example, 40 nucleotides) sequence identical to target, (2) a short stretch (15 nucleotides) of random sequence, and (3) a sequence complementary to the 3′-most 30 nucleotides of MDV-1 RNA. The random sequence element may be generated with ease by addition of all four blocked nucleotides during synthesis of the oligonucleotide, a procedure easily accomplished with currently available automated synthesizers. A second oligonucleotide is then synthesized which is complementary to the nineteen 5′-most nucleotides of the first oligonucleotide. This is annealed to the first oligonucleotide and extended with a DNA-dependent DNA polymerase to produce a double-stranded DNA fragment. This fragment is then cleaved with the restriction enzyme Bst EII and the cleavage product ligated to a fragment of a MDV cDNA clone such as described by Lizardi (supra), previously cleaved with Bst EII. Two picomoles of the ligation product is then transcribed by T7 RNA polymerase to generate a population of RNAs containing 10¹ different spacer sequences.

Molecules within the population of RNAs which most efficiently initiate replication are advantageously selected by one of two methods: electrophoretic retardation of Qβ template RNAs hybridized to their nascent initiated daughter strands (Mills et al. (1980) Biochem 19: 228-236); or isolation of the stable complex formed between Qβ replicase and template RNAs which have directed intiation of a daughter strand. An example of the latter method is described below.

Two picomoles of RNAs bearing random sequence spacers is incubated with 5 picomoles (1.2µg) of Qβ replicase for 5 minutes at 37°C in 25 µl of 100mM Tris HCl, pH7.5, 14mM MgCl₂. Five µl of the same buffer containing 2.4mM each GTP and ATP is added, and the incubation continued for an additional 5 minutes. Five µl of buffer containing 40 picomoles of MDV-1 RNA is then added and the incubation continued for 2 minutes. The reaction is chilled to 0°C and applied to the top of a 2.2ml 10-30% glycerol gradient in 10mM Tris-HCl, pH7.5, 1mM MgCl₂ prechilled to 0°C. The gradient is spun at 55K RPM in a TLS-55 rotor (Beckman Instruments, Palo Alto, CA) for 6 hours at 4°C. The enzyme: RNA complex, which sediments 40% faster than free RNA, is collected, extracted with phenol, and ethanol precipitated. This population is enriched in RNAs which can be replicated efficiently by Qβ replicase. To further enrich this population, a cDNA library of this population is first constructed. An oligonucleotide complementary to the probe region is used to prime synthesis of a single-stranded cDNA population using reverse transcriptase (Maniatis et al., supra). A second oligonucleotide is synthesized to contain, in 5′ to 3′ order, (1) a restriction enzyme sequence to allow cloning of the duplex cDNA, (2) the sequence of a promoter for T7 RNA polymerase, and (3) the sequence of the first 22 nucleotides of MDV-1 RNA. This oligonucleotide and the one used for priming synthesis of the first strand cDNA product are used in a PCR reaction to generate one picomole of duplex DNA using as a template the first strand cDNA. This product is purified by native polyacrylamide gel electrophoresis, and transcribed with T7 RNA polymerase to generate an RNA population which is subjected again to the complex formation-sedimentation protocol. This process may be repeated as many times as desired, but as will be recognized, will converge upon some limiting degree of enrichment. The degree of enrichment after each cycle of selection is determined by cloning the duplex cDNAs into plasmid vectors, and examining the sensitivity of detection of the RNAs produced by transcription of several (for example, 12) such clones chosen at random from the products of each cycle of selection.

The sensitivity of detection of the cloned RNAs may be determined as follows. The plasmid DNA from each clone is purified by standard methods. Each is digested with a restriction enzyme immediately downstream of the cDNA and transcribed by T7 RNA polymerase to produce an RNA product devoid of vector sequence. This RNA is purified either by gel electrophoresis or by capture onto oligo dT-magnetic beads as in the method described in Example 6 with a DNA oligonucleotide of the same sequence as the target tailed with dA₁₅₀, except that a secondary capture probe is omitted. Each of the purified transcripts is serially diluted in water and aliquots (10µl) added to Qβ reactions as described in Example 6. The reactions are allowed to run 30 minutes at 37°C, stopped, and the minimum number of probes required to observe product determined by the highest dilution producing fluorescence. This process is repeated for clones obtained from each round of the selection process.

It will be readily appreciated that the above process may also be applied to the isolation of RNAs which replicate efficiently when hybridized to target. This is simply performed by first hybridizing the population of RNAs to a synthetic target prior to the formation of initiation complexes. As will also be recognized, members of such a population may contain molecules which replicate more efficiently when hybridized to target than when free. Such molecules will be preferred probes for use in hybridization assays since the molecules non-specifically carried through the hybridization process will be intrinsically less efficiently replicated.

### Example 9: A smart probe system for the detection of HIV-1 RNA

An oligonucleotide of the sequence: 5'-AATTCTTTAAAAAATCATAGGACAGGTAA GAGATCAAGCTGAACATCTTGGAGGGAAACAGGACTCTCAGGGAACCCCCCTTCGGGGG-3' is synthesized. A second oligonucleotide of the sequence: 5'-GTGACCCCCCGAAGGGGGGTTCCCTTTTGTCCTGACAGTCCCTCCAAGATGTTCAGCTTGATCTCTTACCTGTCCTATGATTTTTTAAAG-3' is synthesized. The two oligonucleotides are annealed and ligated into a cDNA clone of a recombinant MDV RNA as described by Lizardi (supra) and previously digested with Bst EII and EcoRI. The resultant clone is digested with Dra I and transcribed with T7 RNA polymerase. The product RNA is purified on a 6% polyacrylamide gel containing 8.3M urea, and eluted from the gel after visualization either by autoradiography or UV shadowing. A template oligonucleotide having the sequence: 5'-TACCAGGTAATATACCACAACGTGTGTTTCTCTGCTTGACTTCTC TGTTTGGGGGGGAGACAGCAGTACAAATGGCAGTATTCATCCACAATTTTCCCTATAGTGAGTCGTATT AAT-3' for a ribozyme-probe is synthesized. Two picomoles of this product is annealed to an equimolar amount "T7 promoter-primer" oligonucleotide (Promega) and transcribed by T7 RNA polymerase as described by Mulligan (supra), except that α-³P CTP (1 Ci/mmol) is included in the reaction in order to label the product for visualization by autoradiography. The transcription product is purified on a 10% polyacrylamide gel containing 8.3M urea and eluted. One nanogram of each of the above RNAs is added to an HIV-1 assay as described in Example 7. After washing the beads for the final time as described in Example 6, the beads are resuspended in 50µl of an elution buffer containing 200mM KCl, 10mM MgC₁₂, and 0.5% NP-40. The beads are incubated in this buffer at 37°C for 10 minutes, and then recollected by placing in a magnetic field. The liquid phase is collected and transferred to a fresh tube. The RNA present in 10µl of this eluate is amplified in a Qβ reaction as described in Example 6.

It will become readily apparent to those skilled in the art that numerous modification of the foregoing description and examples of probe-template constructs and assay methods, including, for example, probe length changes, selection of alternative midivariants or alternative sequences coordinate with alternative ribozyme-like structures may be made.

## Claims

1. A method of detecting a target nucleic acid having a first and a second nucleic acid sequence in a sample suspected of comprising the target nucleic acid, the method comprising:
(a) where necessary obtaining the target nucleic acid in a single stranded form;
(b) further providing a probe comprising:
(i) an RNA template having a 3' end and a 5' end, and being replicable by an RNA directed RNA polymerase;
(ii) a first nucleic acid probe sequence at or near the 5' end of the RNA template and substantially complementary to the first nucleic acid sequence of the target nucleic acid (first target sequence); and
(iii) a second nucleic acid probe sequence at or near the 3' end of the RNA template and substantially complementary to the second nucleic acid sequence of the target nucleic acid (second target sequence);
(c) contacting the sample with the probe under conditions that allow hybridization to occur between the first and second nucleic acid probe sequences and the first and second target sequences, respectively, to form a ribozyme comprising sequences from the probe;
(d) inducing ribozyme cleavage which releases the RNA template from the target nucleic acid;
(e) allowing the released RNA template to be replicated; and
(f) detecting replicated RNA template as an indication of the presence or absence of the target nucleic acid in the sample.

2. A method of detecting a target nucleic acid having a first and second nucleic acid sequence in a sample suspected of comprising the target nucleic acid, the method comprising:
(a) where necessary obtaining the target nucleic acid in single stranded form;
(b) further providing a first probe comprising:
(i) an RNA template having a 3' end and a 5' end, and being replicable by an RNA directed RNA polymerase; and
(ii) a first nucleic acid probe sequence at or near one end of the RNA template and substantially complementary to the first nucleic acid sequence of the target nucleic acid (first target sequence);
(c) additionally providing a second probe comprising:
(i) a second nucleic acid probe sequence substantially complementary to the second nucleic acid sequence of the target nucleic acid (second target sequence); and
(ii) a third sequence at or near one end of the second probe, and being substantially complementary to a portion of the first probe;
(d) contacting the sample with the first and second probes simultaneously or separately under conditions that allow hybridization to occur between the first and second probe sequences and the first and second target sequences, respectively, and between the first and second probes to form a ribozyme comprising sequences from the first and second nucleic acid probes;
(e) inducing ribozyme cleavage which releases the RNA template from the target nucleic acid;
(f) allowing the released RNA template to be replicated; and
(g) detecting replicated RNA template as an indication of the presence or absence of the target nucleic acid.

3. A method as claimed in claim 1 or 2 wherein cleavage is induced by contacting the ribozyme with a divalent cation which is Mn2+, Mg2+ or Ca2+.

4. A method as claimed in any of claims 1 to 3 wherein the first and second target sequences are separated by a sequence comprising 5'-GAAA-3', the second nucleic acid probe sequence has a length of at least about 4 bases, and the RNA template is a midivariant.

5. A method as claimed in any of claims 1 to 4 further comprising separating unhybridized probe from hybridized probe following contact of the probe and target nucleic acid and before including cleavage.

6. A method as claimed in any of claims 1 to 5 further comprising correlating the level of detected replicated RNA templates with the amount of target nucleic acid.

7. A method as claimed in any of claims 1 to 6 wherein the first nucleic acid probe sequence remains hybridized to the first target sequence while ribozyme cleavage is induced.

8. A probe comprising an RNA template that has a 3' end and a 5' end and is replicable by an RNA-directed RNA polymerase, the probe further comprising first and second nucleic acid probe sequences at, or near, respective ends of the RNA template, the probe adapted so that upon hybridization with a target nucleic acid, a ribozyme is formed, which ribozyme, upon cleavage, releases the RNA template in a replicable form.

9. A probe as claimed in claim 8 wherein the template is MDV-1.

10. A probe as claimed in claim 8 or 9 wherein the RNA directed RNA polymerase is Q-beta replicase.

11. A probe as claimed in any of claims 8 to 10 further comprising a nucleic acid spacer sequence, not specific to a target nucleic acid, located between the 3' end of the RNA template and either of the first and second nucleic acid probe sequences.

12. A probe as claimed in any of claims 8 to 11 for detecting the presence or absence of a target nucleic acid sequence having first and second target sequences, the probe comprising:
(a) a first nucleic acid probe sequence at the 5' end of the RNA template and substantially complementary to the first target nucleic acid sequence; and
(b) a second nucleic acid probe sequence at the 3' end of the RNA template and substantially complementary to the second target nucleic acid sequence.

13. A probe as claimed in claim 12 wherein the first and second target nucleic acid sequences are separated by a sequence comprising 5'-GAAA-3' and/or the second nucleic acid probe sequence has a length of at least about 4 bases.

14. A probe as claimed in any of claims 8 to 13 wherein ribozyme structure cleavage separates the second nucleic acid probe sequence from the RNA template.

15. A kit for detecting the presence or absence of a target nucleic acid sequence comprising:
(a) a first probe comprising an RNA template having a 3' end and a 5' end, the RNA template being replicable by an RNA directed RNA polymerase and having a first nucleic acid probe sequence at or near either its 3' or 5' end, the first nucleic acid probe sequence being substantially complementary to a first nucleic acid sequence of the target nucleic acid;
(b) a second probe having a 3' end and a 5' end and comprising a second nucleic acid probe sequence being substantially complementary to a second nucleic acid sequence of the target nucleic acid, the second probe further comprising a third sequence at or near the end opposite to that of the second nucleic acid probe sequence, the third sequence being substantially complementary to a portion of the first probe; the first and second probes forming a ribozyme when bound to the target, the ribozyme being capable of cleaving the RNA template from the first nucleic acid probe sequence in a replicable form; and
(c) optionally including instructions for using the first and second probes for detecting the target nucleic acid.

16. A kit as claimed in claim 15 wherein the first and second target nucleic acid sequences are separated by a sequence comprising 5'-GAAA-3' and/or the second nucleic acid probe sequence has a length of at least 4 bases.

## Patentansprüche

1. Verfahren zum Nachweis einer Zielnucleinsäure mit einer ersten und zweiten Nucleinsäuresequenz in einer Probe, von der angenommen wird, daß sie die Zielnucleinsäure enthält, umfassend:
(a) den Erhalt der Zielnucleinsäure in einzelsträngiger Form, sofern notwendig;
(b) weiterhin die Bereitstellung einer Sonde, umfassend:
(i) eine RNA-Matrize mit einem 3'-Ende und einem 5'-Ende, die durch eine RNA-gerichtete RNA-Polymerase replizierbar ist;
(ii) eine erste Nucleinsäure-Sondensequenz am oder nahe dem 5'-Ende der RNA-Matrize, die im wesentlichen zu der ersten Nucleinsäuresequenz der Zielnucleinsäure (erste Zielsequenz) komplementär ist; und
(iii) eine zweite Nucleinsäure-Sondensequenz am oder nahe dem 3'-Ende der RNA-Matrize, die im wesentlichen zu der zweiten Nucleinsäuresequenz der Zielnucleinsäure (zweite Zielsequenz) komplementär ist;
(c) Inkontaktbringen der Probe mit der Sonde unter Bedingungen, die das Zustandekommen einer Hybridisierung zwischen der ersten bzw. zweiten Nucleinsäure-sondensequenz und der ersten bzw. zweiten Zielsequenz zur Bildung eines die Sequenzen der Sonde umfassenden Ribozyms erlauben;
(d) Induktion der Ribozym-Spaltung, die die RNA-Matrize von der Zielnucleinsäure freisetzt;
(e) Ermöglichung der Replikation der freigesetzten RNA-Matrize; und
(f) Nachweis der replizierten RNA-Matrize als Anzeichen für die Gegenwart oder Abwesenheit der Zielnucleinsäure in der Probe.

2. Verfahren zum Nachweis einer Zielnucleinsäure mit einer ersten und zweiten Nucleinsäuresequenz in einer Probe, von der angenommen wird, daß sie die Zielnucleinsäure enthält, umfassend:
(a) den Erhalt der Zielnucleinsäure in einzelsträngiger Form, sofern notwendig;
(b) weiterhin die Bereitstellung einer ersten Sonde, umfassend:
(i) eine RNA-Matrize mit einem 3'-Ende und einem 5'-Ende, die durch eine RNA-gerichtete RNA-Polymerase replizierbar ist; und
(ii) eine erste Nucleinsäure-Sondensequenz am oder nahe einem Ende der RNA-Matrize, die im wesentlichen zu der ersten Nucleinsäuresequenz der Zielnucleinsäure (erste Zielsequenz) komplementär ist;
(c) zusätzliche Bereitstellung einer zweiten Sonde, umfassend:
(i) eine zweite Nucleinsäure-Sondensequenz, die im wesentlichen zu der zweiten Nucleinsäuresequenz der Zielnucleinsäure (zweite Zielsequenz) komplementär ist; und
(ii) eine dritte Sequenz am oder nahe einem Ende der zweiten Sonde, die im wesentlichen zu einem Teil der ersten Sonde komplementär ist;
(d) gleichzeitiges oder getrenntes Inkontaktbringen der Probe mit der ersten und zweiten Sonde unter Bedingungen, die das Zustandekommen einer Hybridisierung zwischen der ersten bzw. zweiten Sondensequenz und der ersten bzw. zweiten Zielsequenz und zwischen der ersten und zweiten Sonde zur Bildung eines Sequenzen der ersten und zweiten Nucleinsäuresonde umfassenden Ribozyms erlauben;
(e) Induktion einer Ribozym-Spaltung, die die RNA-Matrize von der Zielnucleinsäure freisetzt;
(f) Ermöglichung der Replikation der freigesetzten RNA-Matrize; und
(g) Nachweis der replizierten RNA-Matrize als Anzeichen für die Gegenwart oder Abwesenheit der Zielnucleinsäure.

3. Verfahren nach Anspruch 1 oder 2, wobei die Spaltung durch das Inkontaktbringen des Ribozyms mit einem divalenten Kation, das Mn⁺, Mg⁺ oder Ca⁺ ist, induziert wird.

4. Verfahren nach einem der Ansprüche 1 bis 3, wobei die erste und zweite Zielsequenz durch eine 5'-GAAA-3' umfassende Sequenz getrennt sind, die zweite Nucleinsäure-Sondensequenz eine Länge von mindestens etwa 4 Basen hat und die RNA-Matrize eine Midivariante ist.

5. Verfahren nach einem der Ansprüche 1 bis 4, weiterhin umfassend die Trennung nicht-hybridisierter Sonde von hybridisierter Sonde im Anschluß an den Kontakt der Sonde mit der Zielnucleinsäure und vor der Spaltung.

6. Verfahren nach einem der Ansprüche 1 bis 5, weiterhin umfassend die Korrelierung des Grades an nachgewiesenen replizierten RNA-Matrizen mit der Menge an Zielnucleinsäure.

7. Verfahren nach einem der Ansprüche 1 bis 6, wobei die erste Nucleinsäure-Sondensequenz mit der ersten Zielsequenz im Zustand der Hybridisierung verbleibt, während die Ribozym-Spaltung induziert wird.

8. Sonde, umfassend eine RNA-Matrize, die ein 3'- und ein 5'-Ende hat und durch eine RNA gerichtete RNA-Polymerase replizierbar ist, weiterhin umfassend eine erste und zweite Nucleinsäure-Sondensequenz an oder nahe den jeweiligen Enden der RNA-Matrize, und so angepaßt, daß nach Hybridisierung mit einer Zielnucleinsäure ein Ribozym gebildet wird, das bei Spaltung die RNA-Matrize in einer replizierbaren Form freisetzt.

9. Sonde nach Anspruch 8, wobei die Matrize MDV-1 ist.

10. Sonde nach Anspruch 8 oder 9, wobei die RNA-gerichtete RNA-Polymerase Q-beta-Replikase ist.

11. Sonde nach einem der Ansprüche 8 bis 10, weiterhin umfassend eine für eine Zielnucleinsäure nicht-spezifische Nucleinsäure-Spacersequenz, die zwischen dem 3'-Ende der RNA-Matrize und entweder der ersten oder zweiten Nucleinsäure-Sondensequenz gelegen ist.

12. Sonde nach einem der Ansprüche 8 bis 11 zum Nachweis der Gegenwart oder Abwesenheit einer Zielnucleinsäuresequenz mit einer ersten und zweiten Zielsequenz, umfassend:
(a) eine erste Nucleinsäure-Sondensequenz am 5'-Ende der RNA-Matrize, die im wesentlichen zu der ersten Zielnucleinsäuresequenz komplementär ist; und
(b) eine zweite Nucleinsäure-Sondensequenz am 3'-Ende der RNA-Matrize, die im wesentlichen zu der zweiten Zielnucleinsäuresequenz komplementär ist.

13. Sonde nach Anspruch 12, bei der die erste und zweite Zielnucleinsäuresequenz durch eine 5'-GAAA-3' umfassende Sequenz getrennt sind und/oder die zweite Nucleinsäure-Sondensequenz eine Länge von mindestens etwa 4 Basen hat.

14. Sonde nach einem der Ansprüche 8 bis 13, bei der die Ribozymstruktur-Spaltung die zweite Nucleinsäure-Sondensequenz von der RNA-Matrize trennt.

15. Kit zum Nachweis der Gegenwart oder Abwesenheit einer Zielnucleinsäuresequenz, umfassend:
(a) eine erste Sonde, umfassend eine RNA-Matrize mit einem 3'- und einem 5'-Ende, die durch eine RNA-gerichtete RNA-Polymerase replizierbar ist und eine erste Nucleinsäure-Sondensequenz am oder nahe ihrem 3'- oder 5'-Ende hat, wobei die erste Nucleinsäure-Sondensequenz im wesentlichen zu einer ersten Nucleinsäuresequenz der Zielnucleinsäure komplementär ist;
(b) eine zweite Sonde mit einem 3'- und einem 5'-Ende, umfassend eine zweite Nucleinsäure-Sondensequenz, die im wesentlichen zu einer zweiten Nucleinsäuresequenz der Zielnucleinsäure komplementär ist, wobei die zweite Sonde weiterhin eine dritte Sequenz am oder nahe dem Ende entgegengesetzt zu dem der zweiten Nucleinsäure-Sondensequenz umfaßt und die dritte Sequenz im wesentlichen zu einem Teil der ersten Sonde komplementär ist; und wobei die erste und zweite Sonde bei Bindung an das Ziel ein Ribozym bilden, das fähig ist, die RNA-Matrize von der ersten Nucleinsäure-Sondensequenz in eine replizierbare Form zu spalten; und
(c) gegebenenfalls Anweisungen für die Verwendung der ersten und zweiten Sonde zum Nachweis der Zielnucleinsäure.

16. Kit nach Anspruch 15, wobei die erste und zweite Zielnucleinsäuresequenz durch eine 5'-GAAA-3' umfassende Sequenz getrennt sind und/oder die zweite Nucleinsäure-Sondensequenz eine Länge von mindestens 4 Basen hat.

## Revendications

1. Procédé de détection d'un acide nucléique cible, ayant une première et une deuxième séquences d'acide nucléique, dans un échantillon supposé comprendre l'acide nucléique cible, le procédé comprenant les étapes consistant à :
(a) obtenir, si nécessaire, l'acide nucléique cible sous forme monocaténaire ;
(b) prévoir en outre une sonde comprenant :
(i) une matrice d'ARN ayant une extrémité 3' et une extrémité 5' et étant réplicable à l'aide d'une ARN polymérase ARN dépendante ;
(ii) une première séquence sonde d'acide nucléique au niveau de, ou à proximité de, l'extrémité 5' de la matrice d'ARN et substantiellement complémentaire de la première séquence d'acide nucléique de l'acide nucléique cible (première séquence cible) ; et
(iii) une deuxième séquence sonde d'acide nucléique au niveau de, ou à proximité de, l'extrémité 3' de la matrice d'ARN et substantiellement complémentaire de la deuxième séquence d'acide nucléique de l'acide nucléique cible (deuxième séquence cible) ;
(c) mettre en contact l'échantillon avec la sonde dans des conditions qui autorisent l'hybridation entre la première et la deuxième séquences sondes d'acide nucléique et la première et la deuxième séquences cibles, respectivement, pour former un ribozyme comprenant des séquences issues de la sonde ;
(d) induire le clivage du ribozyme qui libère la matrice d'ARN de l'acide nucléique cible ;
(e) permettre la réplication de la matrice d'ARN libérée ; et
(f) détecter la matrice d'ARN répliquée comme indice de la présence, ou de l'absence, de l'acide nucléique cible dans l'échantillon.

2. Procédé de détection d'un acide nucléique cible, ayant une première et une deuxième séquences d'acide nucléique, dans un échantillon supposé comprendre l'acide nucléique cible, le procédé comprenant les étapes consistant à :
(a) obtenir, si nécessaire, l'acide nucléique cible sous forme monocaténaire ;
(b) prévoir en outre une première sonde comprenant :
(i) une matrice d'ARN ayant une extrémité 3' et une extrémité 5' et étant réplicable à l'aide d'une ARN polymérase ARN dépendante ; et
(ii) une première séquence sonde d'acide nucléique au niveau, ou à proximité, d'une extrémité de la matrice d'ARN et substantiellement complémentaire de la première séquence d'acide nucléique de l'acide nucléique cible (première séquence cible) ;
(c) prévoir en outre une seconde sonde comprenant :
(i) une deuxième séquence sonde d'acide nucléique substantiellement complémentaire de la deuxième séquence d'acide nucléique de l'acide nucléique cible (deuxième séquence cible) ; et
(ii) une troisième séquence au niveau, ou à proximité, d'une extrémité de sonde et substantiellement complémentaire d'une partie de la première sonde ;
(d) mettre en contact l'échantillon avec la première et la deuxième sondes simultanément, ou séparément, dans des conditions qui autorisent l'hybridation entre la première et la deuxième séquences sondes et la première et la deuxième séquences cibles, respectivement, et entre la première et la deuxième sondes pour former un ribozyme comprenant des séquences issues de la première et de la deuxième sondes d'acide nucléique :
(e) induire le clivage du ribozyme qui libère la matrice d'ARN de l'acide nucléique cible ;
(f) permettre la réplication de la matrice d'ARN libérée ; et
(g) détecter la matrice d'ARN répliquée comme indice de la présence, ou de l'absence, de l'acide nucléique cible.

3. Procédé selon la revendication 1 ou 2, dans lequel le clivage est induit par mise en contact du ribozyme avec un cation divalent qui est Mn⁺, Mg⁺ ou Ca⁺.

4. Procédé selon l'une quelconque des revendications 1 à 3, dans lequel la première et la deuxième séquences cibles sont séparées par une séquence comprenant 5'-GAAA-3', la deuxième séquence sonde d'acide nucléique a une longueur d'au moins environ 4 bases et la matrice d'ARN est un midivariant.

5. Procédé selon l'une quelconque des revendications 1 à 4, comprenant en outre la séparation de la sonde non hybridée de la sonde hybridée suite à la mise en contact de la sonde et de l'acide nucléique cible et avant l'induction du clivage.

6. Procédé selon l'une quelconque des revendications 1 à 5, comprenant en outre l'établissement d'une corrélation entre la concentration de matrices d'ARN répliquées détectées et la quantité d'acide nucléique cible.

7. Procédé selon l'une quelconque des revendications 1 à 6, dans lequel la première séquence sonde d'acide nucléique reste hybridée à la première séquence cible, alors que le clivage du ribozyme est induit.

8. Sonde comprenant une matrice d'ARN qui possède une extrémité 3' et une extrémité 5' et qui est réplicable à l'aide d'une ARN polymérase ARN dépendante, la sonde comprenant en outre une première et une deuxième séquences sondes d'acide nucléique au niveau, ou à proximité, des extrémités respectives de la matrice d'ARN, la sonde étant telle que lors d'une hybridation à un acide nucléique cible, un ribozyme se forme, ce dernier libérant, après clivage, la matrice d'ARN sous une forme réplicable.

9. Sonde selon la revendication 8, dans laquelle la matrice est MDV-1.

10. Sonde selon la revendication 8 ou 9, dans laquelle l'ARN polymérase ARN dépendante est une réplicase Q-béta.

11. Sonde selon l'une quelconque des revendications 8 à 10, comprenant en outre une séquence d'espacement d'acide nucléique, qui n'est pas spécifique d'un acide nucléique cible, située entre l'extrémité 3' de la matrice d'ARN et la première, ou la seconde, séquence sonde d'acide nucléique.

12. Sonde selon l'une quelconque des revendications 8 à 11 pour la détection de la présence, ou de l'absence, d'une séquence d'acide nucléique cible ayant une première et une seconde séquences cibles, la sonde comprenant :
(a) une première séquence sonde d'acide nucléique au niveau de l'extrémité 5' de la matrice d'ARN et substantiellement complémentaire de la première séquence d'acide nucléique cible ; et
(b) une deuxième séquence sonde d'acide nucléique au niveau de l'extrémité 3' de la matrice d'ARN et substantiellement complémentaire de la deuxième séquence d'acide nucléique cible.

13. Sonde selon la revendication 12, dans laquelle la première et la deuxième séquences d'acide nucléique cibles sont séparées par une séquence comprenant 5'-GAAA-3' et/ou la deuxième séquence sonde d'acide nucléique a une longueur d'au moins environ 4 bases.

14. Sonde selon l'une quelconque des revendications 8 à 13, dans laquelle le clivage de la structure ribozymique sépare la deuxième séquence sonde d'acide nucléique de la matrice d'ARN.

15. Coffret pour la détection de la présence, ou de l'absence, d'une séquence d'acide nucléique cible comprenant :
(a) une première sonde comprenant une matrice d'ARN ayant une extrémité 3' et une extrémité 5', la matrice d'ARN étant réplicable à l'aide d'une ARN polymérase ARN dépendante et ayant une première séquence sonde d'acide nucléique au niveau de, ou à proximité de, son extrémité 3', ou de son extrémité 5', la première séquence sonde d'acide nucléique étant substantiellement complémentaire de la première séquence d'acide nucléique de l'acide nucléique cible ;
(b) une seconde sonde ayant une extrémité 3' et une extrémité 5' et comprenant une deuxième séquence sonde d'acide nucléique substantiellement complémentaire d'une deuxième séquence d'acide nucléique de l'acide nucléique cible, la deuxième sonde comprenant en outre une troisième séquence au niveau de, ou à proximité de, l'extrémité opposée à celle de la deuxième séquence sonde d'acide nucléique, la troisième séquence étant substantiellement complémentaire d'une partie de la première sonde ; la première et la deuxième sondes formant un ribozyme lorsqu'elles sont fixées à la cible, le ribozyme étant apte à cliver la matrice d'ARN de la première séquence sonde d'acide nucléique sous une forme réplicable ; et
(c) comprenant éventuellement des instructions d'utilisation de la première et de la deuxième sondes pour la détection de l'acide nucléique cible.

16. Coffret selon la revendication 15, dans lequel la première et la deuxième séquences d'acide nucléique cibles sont séparées par une séquence comprenant 5'-GAAA-3' et/ou la deuxième séquence sonde d'acide nucléique a une longueur d'au moins environ 4 bases.
